# EUROPEAN PATENT SPECIFICATION

(11) **EP 2 968 865 B1**
(45) Date of publication and mention of the grant of the patent: **04.09.2024**
(21) Application number: 14773999.9
(22) Date of filing: 10.03.2014
(51) Int. Cl.: A61M 25/14, A61B 17/32

(54) **THROMBECTOMY CATHETERS**
THROMBEKTOMIEKATHETER
CATHÉTERS DE THROMBECTOMIE

(30) Priority: 14.03.2013 US 201313827208
(43) Date of publication of application: 20.01.2016
(60) Divisional application: 17166128.3
(73) Proprietor: Boston Scientific Medical Device Limited, Galway (IE)
(72) Inventor: BONNETTE, Michael, J., Minneapolis, MN 55408 (US); THOR, Eric, J., Arden Hills, MN 55112 (US); BRONSTAD, Jason, M., St. Francis, MN 55070 (US); SCHROM, Michael, Forest Lake, MN 55025 (US); LEIRFALLOM, Leif, E., Plymouth, MN 55447 (US); KOZAK, Debra, M., Forest Lake, MN 55025 (US)
(74) Representative: Vossius & Partner Patentanwälte Rechtsanwälte mbB
(86) International application number: PCT/US2014/022503
(87) International publication number: WO 2014/159204

(56) References cited:
- WO-A1-90/05493
- US-A- 4 690 672
- US-A- 5 203 777
- US-A- 5 203 777
- US-A1- 2008 300 532
- US-A1- 2011 152 908
- US-B1- 6 285 903
- US-B2- 6 805 684

## Description

Thrombectomy catheters having at least one outflow orifice and one or more inflow orifices, systems including the thrombectomy catheters, and methods of using the thrombectomy catheters are described herein.

Catheters may be used for a variety of procedures. Some catheters, typically referred to as thrombectomy catheters, may be used to perform procedures in which thrombotic material is removed from a blood vessel (or other body lumen). The removed material may preferably be removed from the body through the thrombectomy catheter.

Examples of some thrombectomy catheters are described in, e.g., U.S. Patent Application Publication US 2008/0188831 A1 (MINIATURE FLEXIBLE THROMBECTOMY CATHETER by Bonnette et al.); U.S. Patent No. 6,875,193 (RAPID EXCHANGE FLUID JET THROMBECTOMY DEVICE AND METHOD to Bonnette et al.); U.S. Patent No. 6,805,684 (THROMBECTOMY CATHETER AND SYSTEM to Bonnette et al.); U.S. Patent No. 6,755,803 (SINGLE OPERATOR EXCHANGE FLUID JET THROMBECTOMY DEVICE to Le et al.); U.S. Patent Application Publication US 2006/0064123 A1 (RAPID EXCHANGE FLUID JET THROMBECTOMY DEVICE AND METHOD to Bonnette et al.); and U.S. Patent Application Publication No. US 2007/0129679 (PNEUMATIC DRIVE UNIT to Bonnette et al.).

US 6 805 684 B2 discloses a cross stream thrombectomy catheter and system for fragmentation and removal of thrombus or other material from blood vessels or other body cavities. High velocity saline jets emitted from a toroidal loop jet emanator or other jet emanator in a catheter distal end entrain fluid through inflow orifices, and with flow resistances create a back-pressure which drives cross stream streams through outflow orifices in a radial direction and thence radially and circumferentially to apply normal and drag forces on thrombotic deposits or lesions in the blood vessel or other body cavity, thereby breaking apart and transporting thrombus particles to be entrained through the inflow orifices, whereupon the high velocity jets macerate the thrombus particles which then transit an exhaust lumen or recirculate again via the outflow orifices.

US 2011/152908 A1 discloses a catheter comprising: a catheter body with a catheter lumen extending from a proximal catheter portion to a distal catheter portion; a manifold assembly coupled with the proximal catheter portion, a manifold lumen extends through the manifold assembly from a proximal manifold portion to a distal manifold portion, the manifold lumen is in communication with the catheter lumen, and the manifold lumen includes an assembly cavity extending around the proximal catheter portion; a fluid jet loop coupled with a high pressure tube at the distal catheter portion, the fluid jet loop extends around a fluid jet loop lumen, the fluid jet loop includes fluid jet orifices along a tapered loop guide surface, and the tapered loop guide surface tapers from a fluid jet loop outer perimeter toward a fluid jet loop inner perimeter and the fluid jet loop lumen; a composite guide including: a proximal guide insert positioned within the assembly cavity, the proximal guide insert includes a guide insert surface substantially flush with a catheter body interior wall, and the proximal guide insert is configured to guide an instrument past the assembly cavity and into the catheter lumen, and a distal guide including the tapered loop guide surface and an intermediate guide surface of the catheter lumen flushly engaged with a leading edge of the tapered loop guide surface, wherein the distal guide is configured to guide the instrument over the intermediate guide surface, the tapered loop guide surface and through the fluid jet loop.

### SUMMARY

The invention is defined by the features of the claims. Embodiments not falling within the scope of the claims are not part of the invention.

Thrombectomy catheters having at least one outflow orifice and one or more inflow orifices, systems including the thrombectomy catheters, and methods of using the thrombectomy catheters are described herein.

In one or more embodiments, the thrombectomy catheters described herein may include one or more of the protective features described herein that may limit potential hazards to vessel walls in which the thrombectomy catheters are used.

Among the protective features that may be used in one or more embodiments of the thrombectomy catheters described herein is the use of radiopaque markers on the high-pressure tube which is, itself, located off center within the catheter lumen such that the rotational orientation of the inflow orifice or orifices can be monitored during use of the thrombectomy catheter.

Another protective feature that may be used in one or more embodiments of the thrombectomy catheters described herein is in the location of fluid jet openings in a fluid jet emanator from which fluid jets emanate relative to an inflow orifice in the catheter. In particular, the inflow orifice (or orifices) may extend about a portion of the circumference of the wall of the catheter that is defined by an orifice arc having a center at the center of the catheter lumen. In one or more embodiments, the openings in the fluid jet emanator are located outside of that orifice arc such that fluid jets emanating from those openings are located a distance away from the inflow orifice. As a result, fluid jets emanating from the fluid jet openings are less likely to come into direct contact with tissue that may be drawn into the inflow orifice or orifices of the thrombectomy catheter.

Another protective feature that may be used in one or more embodiments of the thrombectomy catheters described herein is the use of a high-pressure tube located off center within the catheter lumen and which extends across the opening of an inflow orifice in the thrombectomy catheter. In addition, the high-pressure tube may include an arcuate portion which arcs away from a center of the catheter lumen within the inflow orifice. As a result, the high-pressure tube may limit the entry of tissue into the inflow orifice during use of the thrombectomy catheter.

In a first aspect, one or more embodiments of a thrombectomy catheter as described herein may include: a catheter body extending from a distal portion toward a proximal portion along a longitudinal axis: a high pressure tube extending through a lumen of the catheter body from the proximal portion toward the distal portion, the high pressure tube configured for coupling with a fluid source near the proximal portion and terminating in a fluid jet emanator located in the catheter lumen, the fluid jet emanator comprising a plurality of fluid jet openings configured to direct a plurality of fluid jets through the catheter lumen toward the proximal portion, wherein the high pressure tube extends along one side of the catheter lumen such that the high pressure tube is off-center within the catheter lumen; an inflow orifice formed through a wall of the catheter body in the distal portion, wherein the inflow orifice is located proximally from the fluid jet emanator; a radiopaque marker attached to the high pressure tube within an axial length of the inflow orifice as measured along the longitudinal axis, wherein the radiopaque marker is off-center within the catheter lumen; and an outflow orifice in the distal portion, wherein the outflow orifice is located proximally of the inflow orifice such that the inflow orifice is located between the outflow orifice and the fluid jet emanator.

In one or more embodiments of the thrombectomy catheters described herein, the inflow orifice extends about a portion of the circumference of the wall defined by an orifice arc having a center at a center of the catheter lumen within the inflow orifice, and wherein the plurality of fluid jet openings in the fluid jet emanator are located outside of the orifice arc. In one or more embodiments, a portion of the fluid jet emanator within the orifice arc is free of any fluid jet openings.

In one or more embodiments of the thrombectomy catheters described herein, the high pressure tube extends across the inflow orifice. In one or more embodiments, the high pressure tube comprises an arcuate portion located in the inflow orifice, wherein the arcuate portion arcs away from a center of the cadieter lumen within the inflow orifice. In one or more embodiments, the radiopaque marker is attached to the arcuate portion of the high pressure tube.

In one or more embodiments of the thrombectomy catheters described herein, the distal portion of the catheter body between the inflow orifice and the outflow orifice forms an arc when unrestrained, and wherein the inflow orifice and the outflow orifice face a center of the arc.

In a second aspect, one or more embodiments of the thrombectomy catheters described herein may include: a catheter body extending from a distal portion toward a proximal portion along a longitudinal axis; a high pressure tube extending through a lumen of the catheter body from the proximal portion toward the distal portion, the high pressure tube configured for coupling with a fluid source near the proximal portion and terminating in a fluid jet emanator located in the catheter lumen, the fluid jet emanator comprising a plurality of fluid jet openings configured to direct a plurality of fluid jets through the catheter lumen toward the proximal portion: an inflow orifice formed through a wall of the catheter body in the distal portion, wherein the inflow orifice is located proximally from the fluid jet emanator, and wherein the inflow orifice extends about a portion of the circumference of the wall defined by an orifice arc having a center at a center of the catheter lumen within the inflow orifice, and wherein the plurality of fluid jets emanating from the fluid jet emanator are located outside of the orifice arc; and an outflow orifice in the distal portion, wherein the outflow orifice is located proximally of the inflow orifice such that the inflow orifice is located between the outflow orifice and the fluid jet emanator.

In one or more embodiments of the thrombectomy catheters described herein, a portion of the fluid jet emanator within the orifice arc is free of any fluid jet openings.

In one or more embodiments of the thrombectomy catheters described herein, the high pressure tube is located off-center within the catheter lumen and extends across the inflow orifice. In one or more embodiments, the high pressure tube comprises an arcuate portion located in the inflow orifice, wherein the arcuate portion arcs away from a center of the catheter lumen within the inflow orifice. In one or more embodiments, a radiopaque marker is attached to the arcuate portion of the high pressure tube.

In one or more embodiments of the thrombectomy catheters described herein, the distal portion of the catheter body between the inflow orifice and the outflow orifice forms an arc when unrestrained, and wherein the inflow orifice and the outflow orifice face a center of the arc.

In a third aspect, one or more embodiments of a thrombectomy catheter as described herein may include: a catheter body extending from a distal portion toward a proximal portion along a longitudinal axis; an inflow orifice formed through a wall of the catheter body in the distal portion; a high pressure tube extending through a lumen of the catheter body from the proximal portion toward the distal portion, the high pressure tube configured for coupling with a fluid source near the proximal portion and terminating in a fluid jet emanator located in the catheter lumen, the fluid jet emanator comprising a plurality of fluid jet openings configured to direct a plurality of fluid jets through the catheter lumen toward the proximal portion, wherein the fluid jet emanator is located distal from the inflow orifice, and wherein the high pressure tube is located off center within the catheter lumen and extends across the inflow orifice, and further wherein the high pressure tube comprises an arcuate portion located in the inflow orifice, wherein the arcuate portion arcs away from a center of the catheter lumen within the inflow orifice; and an outflow orifice in the distal portion, wherein the outflow orifice is located proximally of the inflow orifice such that the inflow orifice is located between the outflow orifice and the fluid jet emanator.

In one or more embodiments of the thrombectomy catheters described herein, the distal portion of the catheter body between the inflow orifice and the outflow orifice forms an arc when unrestrained, and wherein the inflow orifice and the outflow orifice face a center of the arc. In one or more embodiments, a radiopaque marker is attached to the arcuate portion of the high pressure tube.

In a fourth aspect, one or more embodiments of the thrombectomy catheters described herein may include: a catheter body extending from a distal portion toward a proximal portion along a longitudinal axis; a high pressure tube extending through a lumen of the catheter body from the proximal portion toward the distal portion, the high pressure tube configured for coupling with a fluid source near the proximal portion and terminating in a fluid jet emanator located in the catheter lumen, the fluid jet emanator comprising a plurality of fluid jet openings configured to direct a plurality of fluid jets through the catheter lumen toward the proximal portion: an inflow orifice formed through a wall of the catheter body in the distal portion, wherein the inflow orifice is located proximally from the fluid jet emanator; and an outflow orifice in the distal portion, wherein the outflow orifice is located proximally of the inflow orifice such that the inflow orifice is located between the outflow orifice and die fluid jet emanator; wherein the distal portion of the catheter body between the inflow orifice and the outflow orifice forms an arc when unrestrained, and wherein the inflow orifice and the outflow orifice face a center of the arc.

In one or more embodiments of the thrombectomy catheters described herein, the high pressure tube is located off-center within the catheter lumen and extends across the inflow orifice, wherein the high pressure tube comprises an arcuate portion located in the inflow orifice, wherein the arcuate portion arcs away from a center of the catheter lumen within the inflow orifice, and wherein a radiopaque marker is attached to the arcuate portion of the high pressure tube.

In a fifth aspect, one or more embodiments of a thrombectomy catheter system as described herein may include: a manifold comprising a high pressure connection branch, an exhaust branch, and a catheter branch, wherein the manifold comprises visible reference indicia proximate the catheter branch and a thrombectomy catheter. The thrombectomy catheter may include: a rotating fitting configured for rotatable connection to the catheter connection branch of the manifold such that rotation of the rotating fitting rotates the thrombectomy catheter relative to the visible reference indicia on the manifold, wherein the rotating fitting comprises visible indicia proximate the visible reference indicia on the manifold when the rotating fitting is connected to the catheter connection branch, and wherein the visible indicia on the rotating fitting is configured to provide an indication of rotational orientation of the thrombectomy catheter relative to the visible reference indicia on the manifold; a catheter body attached to rotating fitting, the catheter body comprising a proximal portion extending away from the rotating fitting towards a distal portion along a longitudinal axis; a high pressure tube extending through the high pressure connection branch and into a lumen of the catheter body from the proximal portion toward the distal portion, the high pressure tube configured for coupling with a fluid source and terminating in a fluid jet emanator located in the catheter lumen, the fluid jet emanator comprising a plurality of fluid jet openings configured to direct a plurality of fluid jets through the catheter lumen toward the proximal portion; an inflow orifice formed through a wall of the catheter body in the distal portion, wherein the inflow orifice is located proximally from the fluid jet emanator; and an outflow orifice in the distal portion, wherein the outflow orifice is located proximally of the inflow orifice such that the inflow orifice is located between the outflow orifice and the fluid jet emanator.

In one or more embodiments of the thrombectomy catheter systems described herein, the thrombectomy catheter comprises a radiopaque marker attached to the high pressure tube within an axial length of the inflow orifice as measured along the longitudinal axis. In one or more embodiments, the radiopaque marker is off-center within the catheter lumen.

In one or more embodiments of the thrombectomy catheter systems described herein, the inflow orifice extends about a portion of the circumference of the wall defined by an orifice arc having a center at a center of the catheter lumen within the inflow orifice, and wherein the plurality of fluid jet openings in the fluid jet emanator are located outside of the orifice arc. In one or more embodiments, a portion of the fluid jet emanator within the orifice arc is free of any fluid jet openings.

In one or more embodiments of the thrombectomy catheter systems described herein, the high pressure tube extends across the inflow orifice. In one or more embodiments, the thrombectomy catheter comprises a radiopaque marker attached to the high pressure tube within an axial length of the inflow orifice as measured along the longitudinal axis. In one or more embodiments, the high pressure tube comprises an arcuate portion located in the inflow orifice, wherein the arcuate portion arcs away from a center of the catheter lumen within the inflow orifice. In one or more embodiments, the thrombectomy catheter comprises a radiopaque marker attached to the high pressure tube within an axial length of the inflow orifice as measured along the longitudinal axis.

In one or more embodiments of the thrombectomy catheter systems described herein, the distal portion of the catheter body between the inflow orifice and the outflow orifice forms an arc when unrestrained, and wherein the inflow orifice and the outflow orifice face a center of the arc.

If used herein, the words "preferred" and "preferably" refer to embodiments described herein that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

As used herein and in the appended claims, the singular forms "a," "an," and "the" include plural referents unless the context clearly dictates otherwise. Thus, for example, reference to "a" or "the" component may include one or more of the components and equivalents thereof known to those skilled in the art. Further, the term "and/or" means one or all of the listed elements or a combination of any two or more of the listed elements.

It is noted that the terms "comprises" and variations thereof do not have a limiting meaning where these terms appear in the accompanying description. Moreover, "a," "an," "the," "at least one," and "one or more" are used interchangeably herein.

Relative terms such as above, below, left, right, forward, rearward, top, bottom, side, upper, lower, horizontal, vertical, and the like may be used herein and, if so, are from the perspective observed in the particular figure. Any such terms should not be used to limit the scope of the inventions described herein unless explicitly indicated otherwise.

The above summary is not intended to describe each embodiment or every implementation of the dust collectors and related methods as described herein. Rather, a more complete understanding of the invention will become apparent and appreciated by reference to the following Description of Illustrative Embodiments and claims in view of the accompanying figures of the drawing.

### BRIEF DESCRIPTIONS OF THE FIGURES OF THE DRAWING

FIG. 1 is a perspective view of one illustrative embodiment of a thrombectomy catheter and manifold as described herein.
FIG. 2 is a perspective view of a portion of a distal portion of one illustrative embodiment of a catheter as described herein.
FIG. 3 is a cross-sectional view of the distal portion of the catheter depicted in FIG. 2 taken along line 3-3 in FIG. 2.
FIG. 4 is a cross-sectional view of the distal portion of the catheter depicted in FIG. 3 taken along line 4-4 in FIG. 3.
FIG. 5 is a perspective view of a portion of a distal portion of another illustrative embodiment of a catheter as described herein.
FIG. 6 is a perspective view of a portion of a distal portion of another illustrative embodiment of a catheter as described herein.
FIG. 7 is a cross-sectional view of the distal portion of the catheter depicted in FIG. 6 taken along line 7-7 in FIG. 6.
FIG. 8 is a cross-sectional view of the distal portion of the catheter depicted in FIG. 7 taken along line 8-8 in FIG. 7.
FIG. 9 is a perspective view of a portion of a distal portion of another illustrative embodiment of a catheter as described herein.
FIG. 10 is a cross-sectional view of one illustrative embodiment of a manifold that may be used with the thrombectomy catheters described herein.
FIG. 11 is an enlarged view of one illustrative embodiment of a junction between a rotating fitting and a manifold body including visible indicia indicative of rotational orientation as described herein.

### DESCRIPTION OF ILLUSTRATIVE EMBODIMENTS

In the following description of illustrative embodiments, reference is made to the accompanying figures of the drawing which form a part hereof, and in which are shown, by way of illustration, specific embodiments. It is to be understood that other embodiments may be utilized and structural changes may be made without departing from the scope of the present invention.

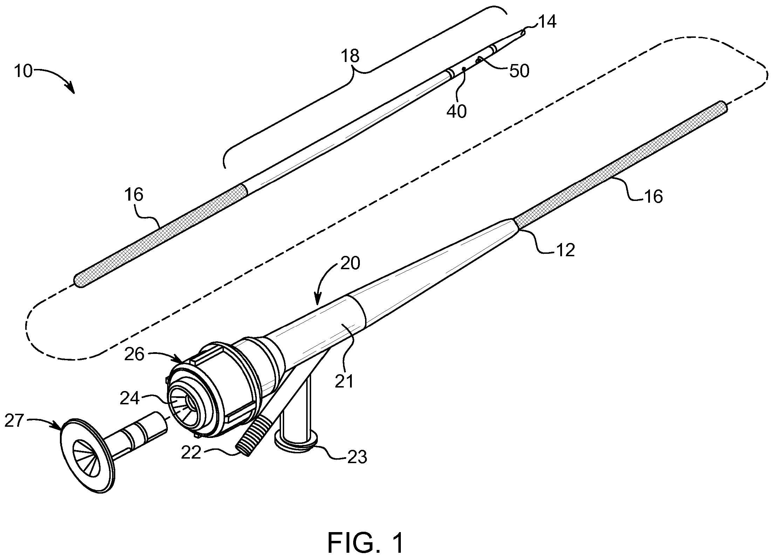

FIG. 1 is a perspective view of components that may be provided in a system that includes a catheter 10 as described herein. Illustrative embodiments of many of these system components may be described in, e.g., US Patent No. 8,162,877 (ENHANCED CROSS STREAM MECHANICAL THROMBECTOMY CATHETER to Bonnette et al.); U.S. Patent No. 6,805,684 (THROMBECTOMY CATHETER AND SYSTEM to Bonnette et al.); and U.S. Patent Application Publication No. US 2007/0129679 (PNEUMATIC DRIVE UNIT to Bonnette et al.).

Among the components in the illustrative embodiment depicted in FIG. 1 is a manifold 20 that is attached to a proximal end 12 of a catheter 10. The manifold 20 may, in one or more embodiments, include a catheter connection branch 21, a high pressure connection branch 22, an exhaust branch 23, and a guidewire port 24, with a hemostatic nut 26 connected to the manifold 20 over the guidewire port 24 and an introducer 27 extending through the nut 26 to secure, e.g., a guidewire (not shown) that may pass into the guidewire port 24.

Although not depicted in FIG. 1, a high pressure fluid source and a high pressure fluid pump may be connected to the manifold 20 via the high pressure connection branch 22 to supply high pressure saline or other suitable fluid to a high pressure tube that extends from the high pressure connection branch 22 into the catheter 10. Examples of some suitable systems including high pressure fluid sources and/or exhaust collection systems may be described in, e.g., US Patent No. 8,162,877 (Bonnette et al.); U.S. Patent No. 6,805,684 (Bonnette et al.); U.S. Patent Application Publication No. US 2007/0129679 (Bonnette et al.); etc.

The catheter 10 may, in one or more embodiments, be constructed of tubular components located between the proximal end 12 and the distal end 14 of the catheter 10. The catheter 10 may, in one or more embodiments, include a proximal portion 16 extending from the proximal end 12 towards the distal end 14 and a distal portion 18 extending from the distal end 14 towards the proximal end 12. The particular location of the junction between the proximal portion 16 and the distal portion 18 may vary between embodiments of catheters as described herein. In one or more embodiments, the proximal portion 16 of the catheter 10 and/or the distal portion 18 of the catheter 10 may be constructed so as to be flexible enough to facilitate advancement of the catheter along a curved passageway such as, e.g., a blood vessel. In one or more embodiments, the distal portion 18 may be, but is not necessarily, shorter than the proximal portion 16. Either or both of the proximal portion 16 and distal portion 18 may include multiple sub-sections having different external sizes. In one or more embodiments, however, the distal portion 18 may have a uniform external size (e.g., diameter for a circular body) over a length between the inflow and outflow orifices in the catheters as described herein.

The tubular components used to manufacture catheters as described herein may, in one or more embodiments, be constructed of materials which promote pushability, torqueability, and which provide for operator feel. In one or more embodiments, the proximal portion 16 may, for example, be constructed of braided polyimide, a synthetic polymeric resin, metal (e.g., stainless steel, Nitinol, etc.) or any other suitable flexible material(s), and the distal portion 18 may be constructed of Pebax, a thermoplastic elastomer, metal (e.g., stainless steel, Nitinol, etc.) or any other suitable material(s). The catheters described herein may, in one or more embodiments, include an external hydrophilic coating to enhance deliverability along the vasculature or other structure. In one or more embodiments, e.g., those including metallic tubing, the tubing may include spiral cuts to improve flexibility.

The external body dimensions of one or more embodiments of the proximal portion 16 and the distal portion 18 may be selected such that the distal portion 18 has smaller external dimensions than the proximal portion 16 which may potentially facilitate advancement of the distal portion 18 into smaller passageways. In one or more embodiments, the proximal portion 16 may have an external dimension of about 4 French (Fr) as measured with reference to the French catheter scale (wherein 1 Fr = 1/3 mm, and 3 Fr = 1 mm). The distal portion 18 may include sub-sections that have different external dimensions. In one or more embodiments, for example, a proximal end of the distal portion 18 can be sized to mate with a distal end of the proximal portion 16 that also has the same diameter. The more distally located subsections of the distal portion 18 may, in one or more embodiments, be drawn and reduced or otherwise processed to reduce their external dimensions relative to the proximal portion to facilitate advancement of the distal portion 18 into smaller passageways. In one or more embodiments, different portions of the thrombectomy catheters described herein may have external dimensions that range from, e.g., 14 French to 3 French, although thrombectomy catheters having external dimensions outside of that range may also be provided.

In one or more embodiments, the proximal portion 16 and the distal portion 18 may be constructed of separate members that are attached to each other by any suitable technique, e.g., the proximal portion 16 and the distal portion 18 can be attached together by adhesive, welding, swaging, or by any other suitable method. In one or more alternative embodiments, the proximal portion 16 and the distal portion 18 may be formed of one continuous member that may be tapered or otherwise reduced in size at one or more transition sections such that joints between separate members may not be required.

Regardless of the specific construction details, the proximal portion 16 and the distal portion 18 may, in one or more embodiments, function together as an exhaust tube for evacuation of material, e.g., macerated effluence from a thrombus or lesion site as described in one or more of the issued patents and/or patent application publications identified herein. To do so, the distal portion 18 includes one or more outflow orifices 40 and one or more inflow orifices 50, the arrangement, construction, and/or use of which are described in more detail herein with respect to various illustrative embodiments.

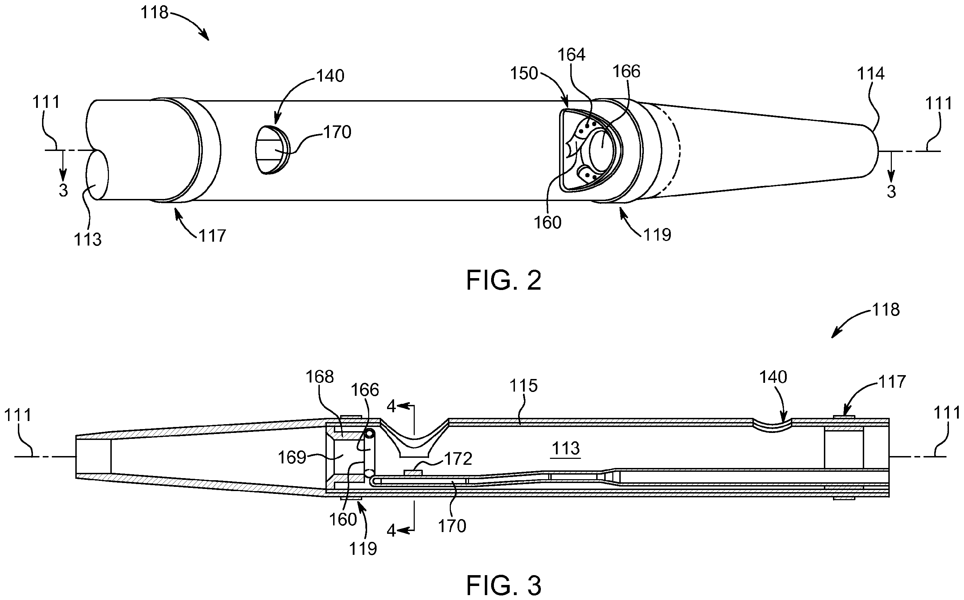

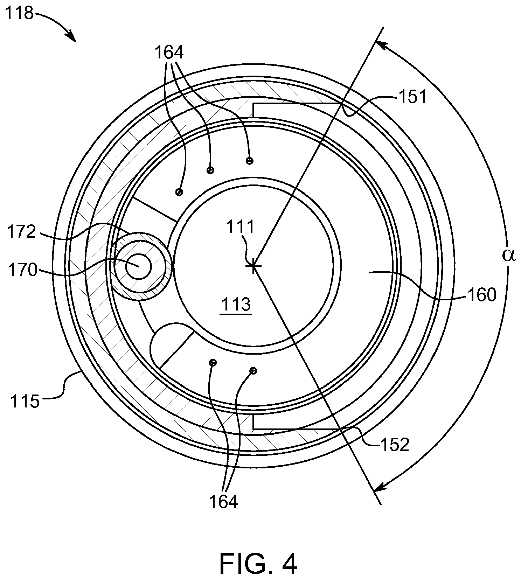

One illustrative embodiment of a distal portion 118 of a catheter as described herein is depicted in FIGS. 2-4. A perspective view of the distal portion 118 of the catheter is provided in FIG. 2, while FIG. 3 is a cross-sectional view of FIG. 2 taken along line 3-3 in FIG. 2 (that is, in the illustrated embodiment, located on the longitudinal axis 111 of the catheter), and FIG. 4 is a cross-sectional view taken along line 4-4 in FIG. 3. In one or more embodiments, the longitudinal axis 111 extends through the catheter from the proximal end (which may, for example, be located in a manifold) to the distal end 114. In the case of a catheter having a circular or generally circular body, the longitudinal axis 111 may be located at the center of the catheter lumen 113.

The distal portion 118 of the catheter as depicted in FIGS. 2 and 3 includes a distal end 114, along with an outflow orifice 140 and an inflow orifice 150. The outflow orifice 140 and the inflow orifice 150 open into the lumen 113 formed within the catheter, with that lumen 113 preferably extending proximally towards a proximal end of the catheter. In one or more embodiments such as the illustrative embodiment of FIGS. 2-4, the outflow orifice 140 and the inflow orifice 150 are aligned with each other along the longitudinal axis 111, i.e., the outflow orifice 140 and the inflow orifice 150 face in the same direction. In one or more alternative embodiments, the outflow orifice 140 and the inflow orifice 150 may not be aligned with each other along the longitudinal axis 111, i.e., the orifices may face in different directions.

The distal portion 118 of the catheter includes optional radiopaque markers 117 and 119 that, in the illustrative embodiment may be positioned such that radiopaque marker 117 is proximal from the outflow orifice 140 and radiopaque marker 119 is located distal from the inflow orifice 150. The radiopaque markers 117 and 119 may be used to assist with monitoring the longitudinal location of the distal portion 118 of the catheter when it is positioned within a vessel or other location using, e.g., fluoroscopic imaging, etc. In particular, placement of the outflow orifice 140 and the inflow orifice 150 may be monitored using the radiopaque markers 117 and 119.

The illustrative embodiment of distal portion 118 of the catheter depicted FIGS. 2-4 includes a fluid jet emanator 160 located at the end of a high pressure tube 170 that, in one or more embodiments, extends proximally through the lumen 113 of the catheter. The depicted illustrative embodiment of fluid jet emanator 160 includes proximally directed fluid jet openings 164 located on the proximal side of the fluid jet emanator 160 for the creation of one or more cross-stream jets outside of the distal portion 118 of the catheter using outflow orifice 140 and inflow orifice 150.

The illustrative embodiment of fluid jet emanator 160 is in the form of an arcuate loop that at least partially defines an opening 166 that may, in one or more embodiments, allow for the passage of a guidewire or other elongate structure through the loop and the lumen 113 to the distal end 114 of the catheter. The fluid jet emanators used in connection with the catheters described herein may take a variety of forms other than a loop in one or more alternative embodiments (i.e., they are not limited to the arcuate loop depicted in FIGS. 2-4). For example, the fluid jet emanators may be in the form of a shorter loop (e.g., a loop that does not extend as far around the interior of the catheter as does loop 160) or other bodies/structures that do not include arcuate tubing (such as, e.g., the fluid jet emanators described in, e.g., U.S. Patent Application Publication US 2006/0064123 A1 (RAPID EXCHANGE FLUID JET THROMBECTOMY DEVICE AND METHOD to Bonnette et al.)).

The fluid jet emanator 160 depicted in FIGS. 2-4 may rest against or otherwise be supported by a support ring 168 located on the distal side of the fluid jet emanator 160. The support ring 168 may, in one or more embodiments, define an opening 169 aligned with the opening 166 in the loop of fluid jet emanator 160 to, e.g., allow a guidewire that is inserted into the distal end 114 of the catheter to pass through the support ring 168.

Although not depicted in FIGS. 2-4, one or more alternative embodiments of catheters as described herein may include a guidewire tube located in the lumen 113, with the guidewire tube extending through one or both of the opening 166 in the fluid jet emanator 160 and opening 169 in the support ring 168. Such a guidewire tube may be used to isolate a guidewire from the fluids within the lumen 113 if such isolation is desired.

Fluid is delivered to the fluid jet emanator 160 through a high-pressure tube 170 that, in the depicted embodiment, passes through lumen 113. In one or more alternative embodiments, the high-pressure tube 170 may be located in a separate lumen that is not a part of the catheter lumen 113. The high-pressure tube 170 extends, in one or more embodiments, from the proximal portion of the catheter to the distal portion 118 of the catheter as seen in FIGS. 2-4. As discussed in the patents identified herein, the high-pressure tube 170 is configured for coupling with a fluid source somewhere within the proximal portion of the catheter. Examples of some potentially suitable connections between a high pressure tube and a fluid source/pump may be found in the patents identified herein.

Also depicted in connection with the illustrative embodiment of FIGS. 2-4 is a radiopaque marker 172 attached to the high-pressure tube 170. The radiopaque marker 172 is, in one or more embodiments, located within the axial length of the inflow orifice 150 as measured along the longitudinal axis 111. As a result, monitoring the position of the radiopaque marker 172 may, in one or more embodiments, provide a user with an indication of the location of the inflow orifice 150 along the longitudinal axis 111 during use of the catheter.

In one or more embodiments, the radiopaque marker 172 is located off-center within the catheter lumen 113. Because the radiopaque marker 172 is located off-center within the catheter lumen 113, monitoring the position of the radiopaque marker 172 also may provide a user with an indication of the rotational orientation of the distal portion 118 of the catheter with respect to the longitudinal axis 111. In one or more embodiments, such as that depicted in FIGS. 2-4, the high-pressure tube 170 is located off-center within the catheter lumen 113 as well (as best seen in, e.g., FIGS. 3-4).

By providing an indication of the rotational orientation of the distal portion 118 of the catheter, the radiopaque marker 172 also provides an indication of the rotational orientation of the inflow orifice 152. As a result, a user may, in one or more embodiments, be able to monitor the direction in which the inflow orifice 150 opens within a vessel as the catheter is being used.

In addition to the features described above which are depicted in connection with the illustrative embodiment of FIGS. 2-4, another feature that may be provided in one or more embodiments of the catheters as described herein may be discussed best with respect to the cross-sectional view of FIG. 4. As seen there, the inflow orifice 150 may extend about an arcuate portion of the circumference of the wall 115 of the catheter between edges 151 and 152 of the inflow orifice 150. The inflow orifice 150 may be described as defining an orifice arc between edges 151 and 152 that which has a center at the center of the catheter lumen 113. The orifice arc as seen in FIG. 4 as an angle α (alpha), which may, in one or more embodiments, occupy a selected portion of the circumference of the catheter. In one or more embodiments, the angle α may have an upper limit of, e.g., about 220 degrees or less. In one or more embodiments, the angle α may have a lower limit of, e.g., about 70 degrees or more. In one or more alternative embodiments, the angle α may have an upper limit of, e.g., about 340 degrees or less, about 300 degrees or less, about 260 degrees or less, or about 180 degrees or less. In one or more alternative embodiments, the angle α may have a lower limit of, e.g., about 30 degrees or more or about 50 degrees or more.

In the illustrative embodiment depicted in FIG. 4, the fluid jet openings 164 in the fluid jet emanator 160 may be located outside of the orifice arc defined by angle α (alpha). Positioning the fluid jet openings 164 in the fluid jet emanator 160 outside of the orifice arc may, in one or more embodiments, reduce the likelihood that a fluid jets emanating from the fluid jet openings 164 will come into direct contact with tissue that may be drawn into the catheter lumen 113 through inflow orifice 150. In another manner of describing the arrangement of fluid jet openings 164 relative to the orifice arc defined by angle α (alpha), the portion of the fluid jet emanator 160 located within the orifice arc may be described as being free of any fluid jet openings 164 provided in the fluid jet emanator 160. In one or more alternative embodiments, however, the fluid jet openings 164 in the fluid jet emanator 160 may be located within the orifice arc.

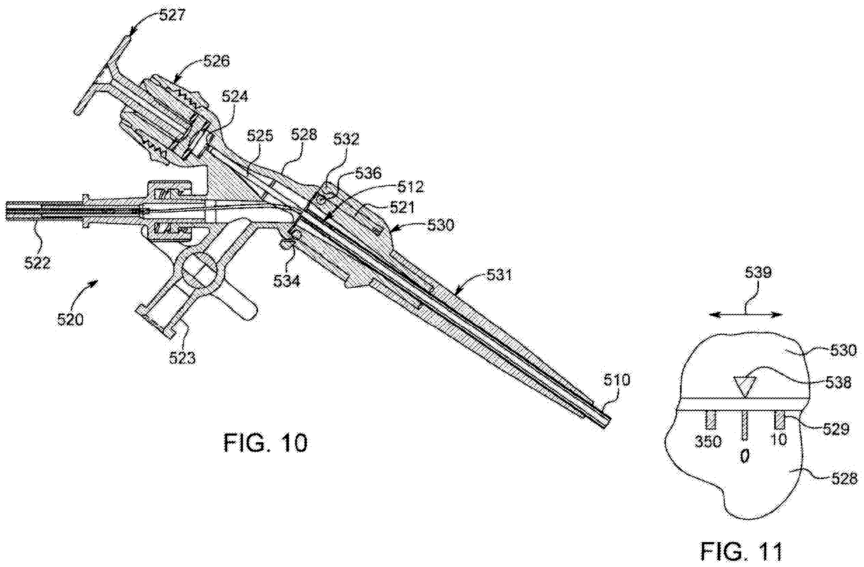

The arrangement of the features in the distal portion 118 of the catheter as depicted in FIGS. 2-4, including, e.g., the outflow orifice 140, inflow orifice 150, fluid jet emanator 160, and catheter lumen 113, may provide a catheter that, in one or more embodiments, produces non-hemolyzing cross-stream jets of saline or other suitable fluids which exit from the outflow orifice 140 to accomplish thrombectomy functions as described in, e.g., US Patent No. 8,162,877 (ENHANCED CROSS STREAM MECHANICAL THROMBECTOMY CATHETER to Bonnette et al.) (see, e.g., FIGS. 10-11 and the corresponding description of that document) and at least some of the other patents identified herein. As discussed there, cross stream jets emanating from one or more outflow orifices may provide for the fluid jet impingement on the deposits of thrombus or lesions on the inner wall of a blood vessel adjacent to or in close proximity to the outflow orifices to impinge, ablate and loosen deposits of thrombus or lesions, whereby such thrombus or lesion particulate and fluids can be drawn into the catheter lumen through one or more inflow orifices for removal proximally through the catheter.

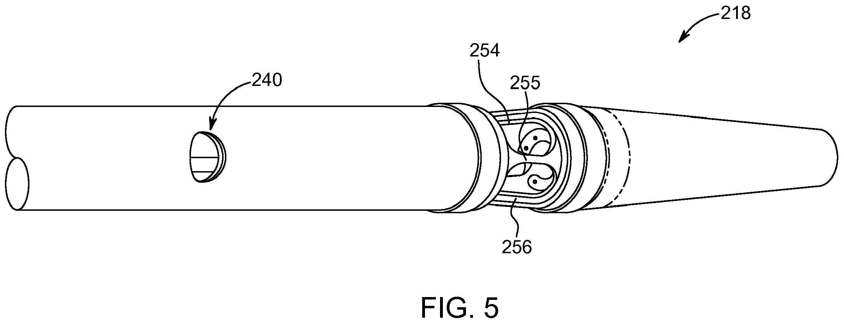

Although the illustrative embodiment depicted in FIGS. 2-4 includes only a single inflow orifice 150, one or more alternative embodiments may include two or more inflow orifices. One illustrative embodiment of a catheter including two inflow orifices is dictated in the perspective view of FIG. 5, wherein the distal portion 218 of the depicted catheter includes a first inflow orifice 254 and a second inflow orifice 256 which are separated from each other by a saddle 255. The distal portion 218 of the depicted catheter also includes a single outflow orifice 240 that may be paired with the two or more inflow orifices of this illustrative embodiment.

Although the catheter depicted in FIGS. 2-4 includes a single inflow orifice 150, an orifice arc may also be defined by two or more circumferentially arranged inflow orifices such as, e.g., orifices 254 and 256 in the distal portion 218 of the catheter as depicted in FIG. 5. In such an embodiment, the orifice arc may be defined between the outermost edges of the inflow orifices 254 and 256.

In one or more embodiments, drugs for treatment or for lysing of the thrombus or lesions on a vessel wall may be delivered via one or more outflow orifices to, e.g., soften the deposits of thrombus or lesions in the region of the blood vessel adjacent to or in close proximity to the outflow orifices. Any such drugs may, in one or more embodiments, be delivered through a high-pressure tube delivering fluid to a fluid jet emanator, through a separate lumen, using a different fluid delivery device (e.g., catheter, etc.).

The sizing of the various components used in connection with one or more embodiments of the systems and methods described herein may vary based on a number of factors such as, e.g., size of the catheter, materials to be removed using the catheter, fluid flow rates desired, etc. In general, however, fluid jet openings in the fluid jet emanators used in one or more embodiments of the catheters described herein can range in size, e.g., from about 0.0254 mm to about 1.016 mm (0.001 inch to about 0.040 inch) for emanation of saline or other suitable fluid therefrom in a velocity range of about 1 to about 250 m/s. By sizing the fluid jet openings and adjusting the high pressure fluid pump, the velocity and strength of the cross stream fluid flow in one or more embodiments can be controlled. The general operating pressures of the catheter systems described herein may, in one or more embodiments, for example, range from about 50 psi to about 20,000 psi (1 psi is approximately 6,895 pascals).

The high pressure tubes used in one or more embodiments of catheters as described herein may, in one embodiment, be circular tubes with an outside diameter of about 0.4572 mm (about 0.018 inch) and in inside diameter of about 0.3048 mm (about 0.012 inch) over a proximal portion, and an outside diameter of about 0.2794 mm (about 0.011 inch) and an inside diameter of about 0.2032 mm (about 0.008 inch) over an intermediate portion, and an outside diameter of about 0.1778 mm (about 0.007 inch) and an inside diameter of about 0.1016 mm (about 0.004 inch) over a distal portion, although other tube profiles and/or dimensions may be used.

The different portions of the catheters described herein may have a variety of different tubular profiles and/or dimensions, although in one or more embodiments, the proximal and distal portions may be circular in profile and have an outside diameter ranging from an upper limit of, e.g., about 14 French to a lower limit of about 3 French, although catheters having different profiles and/or dimensions outside of these upper and/or lower limits may also be provided in one or more alternative embodiments.

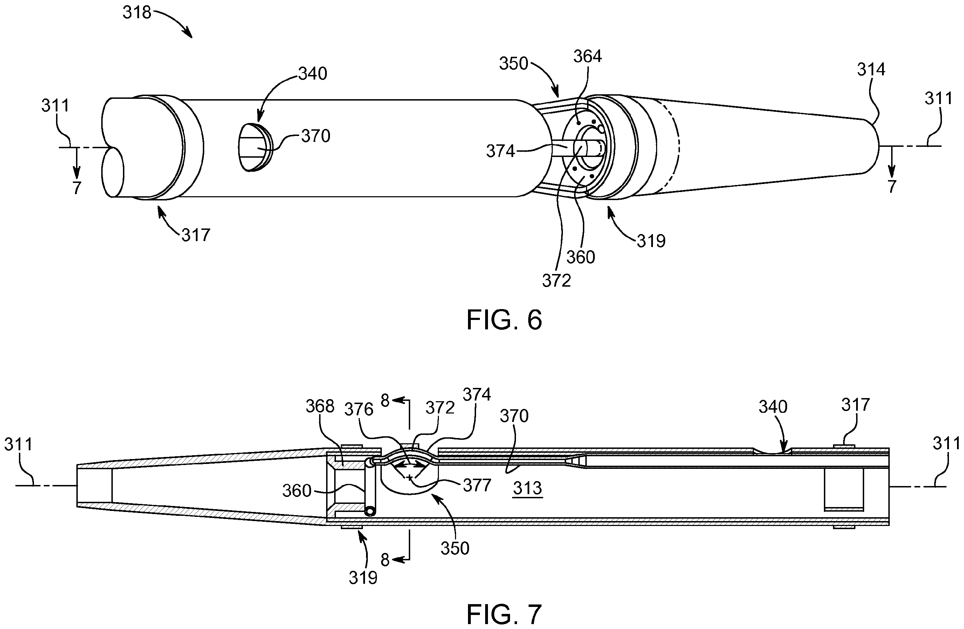

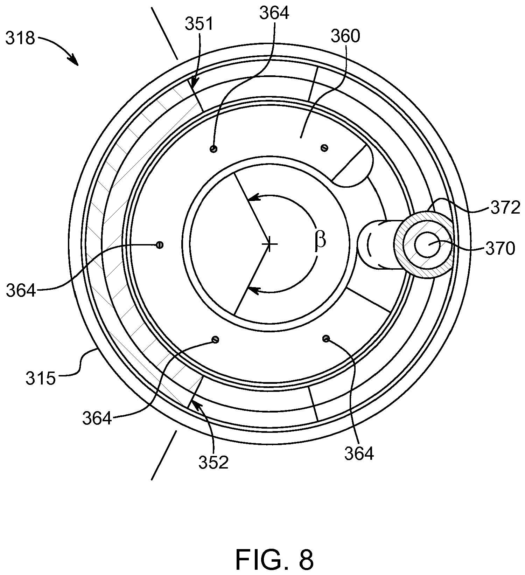

Another illustrative embodiment of a catheter as described herein is depicted in connection with FIGS. 6-8. A perspective view of the distal portion 318 of the catheter is provided in FIG. 6, while FIG. 7 is a cross-sectional view of FIG. 6 taken along line 7-7 in FIG. 6 (that is, in the illustrated embodiment, located on the longitudinal axis 111 of the catheter), and FIG. 8 is a cross-sectional view taken along line 8-8 in FIG. 7. In one or more embodiments, the longitudinal axis 311 extends through the catheter from the proximal end (which may, for example, be located in a manifold) to the distal end 314. In the case of a catheter having a circular or generally circular body, the longitudinal axis 311 may be located at the center of the catheter lumen 313.

The distal portion 318 of the catheter as depicted in FIGS. 6 and 7 includes a distal end 314, along with an outflow orifice 340 and an inflow orifice 350. The inflow orifice 340 and the outflow orifice 350 open into the lumen 313 formed within the catheter, with that lumen 313 preferably extending proximally towards a proximal end of the catheter.

The distal portion 318 of the catheter includes optional radiopaque markers 317 and 319 that, in the illustrative embodiment may be positions such that radiopaque marker 317 is proximal from the outflow orifice 340 and radiopaque marker 319 is located distal from the inflow orifice 350. The radiopaque markers 317 and 319 may be used to assist with monitoring the longitudinal location of the distal portion 318 of the catheter when it is positioned within a vessel or other location using, e.g., fluoroscopic imaging, etc. In particular, placement of the outflow orifice 340 and the inflow orifice 350 may be monitored using the radiopaque markers 317 and 319.

The illustrative embodiment of distal portion 318 of the catheter depicted FIGS. 6-8 includes a fluid jet emanator 360 located at the end of a high pressure tube 370 that, in one or more embodiments, extends proximally through the catheter. The depicted illustrative embodiment of fluid jet emanator 360 includes proximally directed fluid jet openings 364 located on the proximal side of the fluid jet emanator 360 for the creation of a cross-stream jet outside of the distal portion 318 of the catheter using outflow orifice 340 and inflow orifice 350. The fluid jet emanator 360 depicted in FIGS. 6-8 may rest against or otherwise be supported by a support ring 368 located on the distal side of the fluid jet emanator 360.

Fluid is delivered to the fluid jet emanator 360 through a high-pressure tube 370 that, in the depicted embodiment, passes through lumen 313. The high-pressure tube 370 extends, in one or more embodiments, from the proximal portion of the catheter to the distal portion 318 of the catheter as seen in FIGS. 6-8. As discussed in the patents identified herein, the high-pressure tube 370 is configured for coupling with a fluid source near the proximal portion located near the proximal end of the catheter.

Unlike the high-pressure tube 170 depicted in connection with the illustrative embodiment of FIGS. 2-4, the high-pressure tube 370 is positioned within the catheter lumen 313 such that a portion of the high-pressure tube 370 extends across the inflow orifice 350 in the distal portion 318. Although the portion of the high-pressure tube 370 extending across the inflow orifice 350 is depicted as generally bisecting the inflow orifice 350 over its circumferential direction (i.e., from edge 351 to edge 352 as seen in the cross-sectional view of FIG. 8), such an arrangement is not necessarily required. For example, the portion of the high-pressure tube 370 extending across the inflow orifice 350 may, in one or more embodiments, be closer to one edge 351 than the opposite edge 352 (or vice versa).

The portion of high-pressure tube 370 extending across the inflow orifice 350 is, in the depicted embodiment, generally aligned with the longitudinal axis 311, although it should be understood that such an alignment is not necessarily required and that, for example, in one or more alternative embodiments the portion of high-pressure tube 370 extending across the inflow orifice 350 may be oriented in a direction that is not aligned with the longitudinal axis 311.

As best seen in, e.g., the cross-sectional view of FIG. 7, the portion of the high-pressure tube 370 extending across the inflow orifice 350 may include an arcuate portion 374 which arcs away from a center of the catheter lumen 313 within the boundaries of the inflow orifice 350. In one or more embodiments, the center 377 of the arc 376 formed by the arcuate portion 374 of the high-pressure tube 370 may be located on the longitudinal axis 311 where the longitudinal axis extends along the center of the catheter lumen 313, although such an arrangement is not necessarily required. In one or more embodiments in which a high-pressure tube 370 includes an arcuate portion 374 extending across the inflow orifice 350, the arcuate portion 374 of the high-pressure tube 370 may help to limit the entry of tissue into the inflow orifice 350 during operation of the catheter.

Also depicted in connection with the illustrative embodiment of FIGS. 6-8 is a radiopaque marker 372 attached to the high-pressure tube 370. The radiopaque marker 372 may, in one or more embodiments, be located within the arcuate portion 374 of the high-pressure tube 370 which, in the depicted embodiment is also within the axial length of the inflow orifice 350 as measured along the longitudinal axis 311. As a result, monitoring the position of the radiopaque marker 372 may, in one or more embodiments, provide a user with an indication of the location of the inflow orifice 350 along the longitudinal axis 311 during use of the catheter.

In one or more embodiments, the radiopaque marker 372 may also be described as being located off-center within the catheter lumen 313. Because the radiopaque marker 372 is located off-center within the catheter lumen 313, monitoring the position of the radiopaque marker 372 also may provide a user with an indication of the rotational orientation of the distal portion 318 of the catheter with respect to the longitudinal axis 311. By providing an indication of the rotational orientation of the distal portion 318 of the catheter, the radiopaque marker 372 also provides an indication of the rotational orientation of the inflow orifice 350. As a result, a user may, in one or more embodiments, be able to monitor the direction in which the inflow orifice 350 opens within a vessel as the catheter is being used.

In addition to the features described above which are depicted in connection with the illustrative embodiment of FIGS. 6-8, another feature that may be provided in one or more embodiments of the catheters as described herein may be discussed best with respect to the cross-sectional view of FIG. 8. As seen there, the inflow orifice 350 may extend about a portion of the circumference of the wall 315 of the catheter from edge 351 to edge 352. The inflow orifice 350 may be described as defining an orifice arc which has a center at the center of the catheter lumen 313. The orifice arc as seen in FIG. 8 as an angle β (beta), which may, in one or more embodiments, occupy a selected portion of the circumference of the catheter. In one or more embodiments, the angle β (beta) may have an upper limit of less than 360 degrees. In one or more embodiments, the angle β (beta) may have an upper limit of about 70 degrees or less. In one or more embodiments, the angle β (beta) may have a lower limit of about 30 degrees or more. In one or more embodiments, the angle β (beta) may have a lower limit of about 180 degrees or more. In one or more embodiments, the angle β (beta) may have a range of about 30 degrees to less than 360 degrees. In one or more embodiments, the angle β (beta) may have a range of about 30 degrees to about 70 degrees. In one or more embodiments, the angle β (beta) may have a range of about 180 degrees to less than 360 degrees. In one or more embodiments, catheters as described herein may have one or more inflow orifices with an angle β (beta) of about 30 degrees to about 70 degrees in combination with one or more inflow orifices with an angle β (beta) of about 180 degrees to less than 360 degrees. Limiting inflow orifice size to the smaller of those angular ranges (i.e., about 30 degrees to about 70 degrees) may, in one or more embodiments, limit the inflow area which may improve safety. Providing inflow orifice sizes in the larger of those angular ranges (i.e., about 180 degrees to less than 360 degrees) may, in one or more embodiments, limit the inflow velocity and/or suction force which may improve safety.

In one or more embodiments, such as that depicted in FIGS. 6-8, the orifice arc defined by angle β (beta) of the inflow orifice 350 may be greater than about 180 degrees at least due in part to the location of the arcuate portion of the high-pressure tube 370 extending across the inflow orifice 350. As discussed herein, location of the high-pressure tube 370 across the inflow orifice 350 may reduce tissue entry into the inflow orifice 350 that would otherwise occur if the high-pressure tube 370 did not extend across the inflow orifice 350.

As with the embodiment depicted in connection with FIGS. 2-4, the arrangement of the features in the distal portion 318 of the catheter as depicted in FIGS. 6-8, including, e.g., the outflow orifice 340, inflow orifice 350, fluid jet emanator 360, and catheter lumen 313, may provide a catheter that, in one or more embodiments, produces non-hemolyzing cross-stream jets of saline or other suitable fluids which exit from the outflow orifices to accomplish thrombectomy functions as described in, e.g., US Patent No. 8,162,877 (ENHANCED CROSS STREAM MECHANICAL THROMBECTOMY CATHETER to Bonnette et al.) (see, e.g., FIGS. 10-11 and the corresponding description of that document) and at least some of the other patents identified herein. As discussed there, cross stream jets emanating from outflow orifices may provide for the fluid jet impingement on the deposits of thrombus or lesions on the inner wall of a blood vessel adjacent to or in close proximity to the outflow orifices to impinge, ablate and loosen deposits of thrombus or lesions, whereby such thrombus or lesion particulate and fluids can be drawn into catheter lumen through one or more inflow orifices for removal proximally through the catheter.

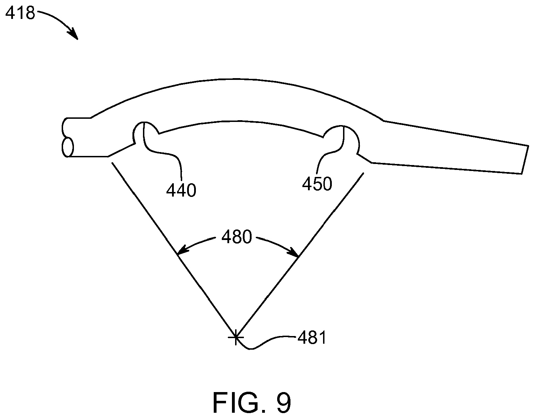

With reference to FIG. 9, another feature that may be included in one or more embodiments of the catheters as described herein is depicted. The distal portion 418 of the catheter as seen in FIG. 9 includes, in a manner similar to the other illustrative embodiments described herein, an outflow orifice 440 and an inflow orifice 450. Other features not depicted in FIG. 9 but described in connection with the other embodiments described herein may be included, such as, e.g., fluid jet emanators, etc. The distal portion 418 of the catheter forms, in the depicted embodiment, an arc 480 between the outflow orifice 440 and the inflow orifice 450 when the distal portion 418 of the catheter is unrestrained. In other words, in the absence of external forces acting on the distal portion 418 of the catheter, the distal portion 418 will form an arc 480 as described herein. The tendency of the distal portion 418 of the catheter to form such an arc may, in one or more embodiments, be overcome through the use of a stylet or other structure, but in the absence of such a structure, the distal portion 418 may take on and arcuate shape between the outflow orifice 440 and the inflow orifice 450.

In one or more embodiments, the outflow orifice 440 and the inflow orifice 450 may face the center 481 of the arc 480 formed by the distal portion 418 of the catheter, an example of which is depicted in FIG. 9. By including a distal portion 418 that forms an arc having a center 401 that is faced by the inflow orifice 450, the likelihood of drawing tissue into the inflow orifice 450 during operation of the catheter may be reduced.

As discussed herein, the distal portions of the catheters described herein may be advantageously used in one or more rotational orientations with respect to a longitudinal axis extending through the catheter. Because the rotational orientation of the distal portions of the catheters may be useful in one or more embodiments, the use of a manifold at the proximal end of the catheter that is capable of providing an indication of the rotational orientation of the distal portion of the catheters may, in one or more embodiments, also be useful.

One illustrative embodiment of a manifold that may be used with the catheters described herein to provide such rotational orientation indications to a user is depicted in FIG. 10. The manifold 520 is, in the depicted embodiment, attached to a proximal end of a catheter 510. The manifold 520 may, in one or more embodiments, include a catheter connection branch 521, a high pressure connection branch 522, and an exhaust branch 523. A guidewire lumen 525 extends through the manifold 520 to a guidewire port 524, with a hemostatic nut 526 connected to the manifold 520 over the guidewire port 524 and an introducer 527 extending through the nut 526 to secure, e.g., a guidewire (not shown) that may be inserted into the guidewire lumen 525 through the guidewire port 524.

Although not depicted in FIG. 10, a high pressure fluid source and a high pressure fluid pump may be connected to the manifold 520 via the high pressure connection branch 522 to supply high pressure saline or other suitable fluid to a high pressure tube 570 that extends from the high pressure connection branch 522 into the catheter 510. In one or more embodiments, the exhaust branch 523 may include a stopcock or other fluid control device to control the flow of fluid out of the manifold 520 through the exhaust branch 523. Examples of some suitable systems including high pressure fluid sources and/or exhaust collection systems may be described in, e.g., US Patent No. 8,162,877 (Bonnette et al.); U.S. Patent No. 6,805,684 (Bonnette et al.); U.S. Patent Application Publication No. US 2007/0129679 (Bonnette et al.); etc.

In one or more embodiments such as the illustrative embodiment depicted in FIG. 10, the proximal end 512 of the catheter 510 is attached to the manifold 520 by the use of a rotating fitting 530 on the catheter branch 521 that allows rotation of the catheter 510 relative to the manifold 520 about a longitudinal axis 511 extending through the catheter 510 while the catheter 510 remains sealed to the manifold 520. That rotation may, in one or more embodiments, be useful in positioning the distal portion (not shown) of the catheter 510 in a desired rotational orientation as described herein. A strain relief tube 531 may, in one or more embodiments, extend distally from the rotating fitting 530 to provide support to the catheter 510 during use of the manifold 520 and attached catheter 510.

In the illustrative embodiment depicted in FIG. 10, the rotating fitting 530 forms a sleeve that is configured to be attached to the catheter branch 521. The fitting 530 is, in one or more embodiments, retained on the catheter branch 521 by a flange 532 that fits within a channel 534 formed in the catheter branch 521. A seal 536 may be provided within the junction between the catheter branch 521 and the fitting to prevent or at least limit leakage out of the manifold 520 through the interface between the catheter branch 521 and the fitting 530. In the depicted illustrative embodiment, the seal 536 is in the form of an O-ring although many other seal constructions may be used in place of an O-ring.

In one or more embodiments, the fitting 530 (which, for the purposes of the discussion herein includes the strain relief tube 531) may include visible markings or some other indicia that indicated the rotational position of features on the catheter 510 (relative to the longitudinal axis 511) that may, themselves, not be visible during use of the catheter 510. Those catheter features may include, e.g., inflow and/or outflow orifices provided in the distal portion of the catheters as described herein. The catheter 510 may, in one or more embodiments, be fixedly attached to the rotating fitting 530 such that rotating of the fitting 530 about the catheter branch 521 causes corresponding rotation of the catheter 510.

FIG. 11 depicts one illustrative embodiment of visible indicia that may be provided on a manifold body 528 (see, e.g., FIG. 10) from which the catheter branch 521 extends and the fitting 530. In particular, fitting 530 may include one or more visible indicia 538 indicative of the rotational orientation of features on the catheter 510 such as, e.g., the inflow and/or outflow orifices in the distal portion of the catheter 510 connected to the fitting 530. A set of reference indicia 529 may be provided on the manifold body 528. Rotation of the fitting 530 about a longitudinal axis extending through the catheter fitting 530 and the attached catheter 510 will move the visible indicia 538 on the fitting 530 in either direction along bidirectional arrow 539 relative to the reference indicia 529 on the manifold body 528, thereby providing a user with an indication of the rotational orientation of the catheter 510 (and features of the catheter 510) relative to the manifold 520. The depicted indicia are illustrative only, i.e., many other indicia may be used in place of those depicted in FIG. 11.

Illustrative embodiments of thrombectomy catheters, systems and methods as described herein are discussed and reference has been made to some possible variations. These and other variations and modifications in the invention will be apparent to those skilled in the art without departing from the scope of the invention, and it should be understood that this invention is not limited to the illustrative embodiments set forth herein. Accordingly, this invention is not limited to the above-described embodiments, but is to be controlled by the limitations set forth in the following claims. This invention also may be suitably practiced in the absence of any element not specifically disclosed as necessary herein.

## Claims

1. A thrombectomy catheter (10; 510) comprising:
a catheter body extending from a distal portion (18; 118; 218; 318; 418) toward a proximal portion (16) along a longitudinal axis (111; 311; 511);
a high pressure tube (170; 370; 570) extending through a lumen of the catheter body from the proximal portion (16) toward the distal portion (18; 118; 218; 318; 418), the high pressure tube (170; 370; 570) configured for coupling with a fluid source near the proximal portion (16) and terminating in a fluid jet emanator (160; 360) located in the catheter lumen (113; 313), the fluid jet emanator (160; 360) comprising a plurality of fluid jet openings (164; 364) configured to direct a plurality of fluid jets through the catheter lumen (113; 313) toward the proximal portion (16), wherein the high pressure tube (170; 370; 570) extends along one side of the catheter lumen (113; 313) such that the high pressure tube (170; 370; 570) is off-center within the catheter lumen (113; 313);
an inflow orifice (50; 150; 254, 256; 350; 450) formed through a wall of the catheter body in the distal portion, wherein the inflow orifice (50; 150; 254, 256; 350; 450) is located proximally from the fluid jet emanator (160; 360);
a radiopaque marker (172; 372) attached to the high pressure tube (170; 370; 570) within an axial length of the inflow orifice (50; 150; 254, 256; 350; 450) as measured along the longitudinal axis, wherein the radiopaque marker (172; 372) is off-center within the catheter lumen (113; 313); and
an outflow orifice (40; 140; 240; 340; 440) in the distal portion, wherein the outflow orifice (40; 140; 240; 340; 440) is located proximally of the inflow orifice (50; 150; 254, 256; 350; 450) such that the inflow orifice (50; 150; 254, 256; 350; 450) is located between the outflow orifice (40; 140; 240; 340; 440) and the fluid jet emanator (160; 360).

2. A catheter (10; 510) according to claim 1, wherein the inflow orifice (50; 150; 254, 256; 350; 450) extends about a portion of the circumference of the wall defined by an orifice arc having a center at a center of the catheter lumen within the inflow orifice (50; 150; 254, 256; 350; 450), and wherein the plurality of fluid jet openings (164; 364) in the fluid jet emanator (160; 360) are located outside of the orifice arc.

3. A catheter (10; 510) according to claim 2, wherein a portion of the fluid jet emanator (160; 360) within the orifice arc is free of any fluid jet openings.

4. A catheter (10; 510) according to claim 1, wherein the high pressure tube (170; 370; 570) extends across the inflow orifice (50; 150; 254, 256; 350; 450).

5. A catheter (10; 510) according to claim 4, wherein the high pressure tube (170; 370; 570) comprises an arcuate portion (374) located in the inflow orifice (50; 150; 254, 256; 350; 450), wherein the arcuate portion (374) arcs away from a center of the catheter lumen (113; 313) within the inflow orifice (50; 150; 254, 256; 350; 450).

6. A catheter (10; 510) according to claim 5, wherein the radiopaque marker (172; 372) is attached to the arcuate portion (374) of the high pressure tube (170; 370; 570).

7. A catheter (10; 510) according to claim 1, wherein the distal portion of the catheter body between the inflow orifice (50; 150; 254, 256; 350; 450) and the outflow orifice (40; 140; 240; 340; 440) forms an arc (480) when unrestrained, and wherein the inflow orifice (50; 150; 254, 256; 350; 450) and the outflow orifice (40; 140; 240; 340; 440) face a center (481) of the arc (480).

## Patentansprüche

1. Thrombektomiekatheter (10; 510), der aufweist:
einen Katheterkörper, der sich von einem distalen Abschnitt (18; 118; 218; 318; 418) zu einem proximalen Abschnitt (16) entlang einer Längsachse (111; 311; 511) erstreckt;
eine Hochdruckleitung (170; 370; 570), die sich durch ein Lumen des Katheterkörpers vom proximalen Abschnitt (16) zum distalen Abschnitt (18; 118; 218; 318; 418) erstreckt, wobei die Hochdruckleitung (170; 370; 570) zur Kopplung mit einer Fluidquelle nahe dem proximalen Abschnitt (16) konfiguriert ist und in einem Fluidstrahlausströmer (160; 360) endet, der sich im Katheterlumen (113; 313) befindet, wobei der Fluidstrahlausströmer (160; 360) mehrere Fluidstrahlöffnungen (164; 364) aufweist, die dazu konfiguriert sind, mehrere Fluidstrahlen durch das Katheterlumen (113; 313) in Richtung proximalen Abschnitt (16) zu leiten, wobei sich die Hochdruckleitung (170; 370; 570) entlang einer Seite des Katheterlumens (113; 313) so erstreckt, dass die Hochdruckleitung (170; 370; 570) im Katheterlumen (113; 313) außermittig angeordnet ist;
eine Einströmdüse (50; 150; 254, 256; 350; 450), die durch eine Wand des Katheterkörpers hindurch im distalen Abschnitt gebildet ist, wobei die Einströmdüse (50; 150; 254, 256; 350; 450) proximal vom Fluidstrahlausströmer (160; 360) angeordnet ist;
einen röntgendichten Marker (172; 372), der an der Hochdruckleitung (170; 370; 570) in einer Axiallänge der Einströmdüse (50; 150; 254, 256; 350; 450), gemessen entlang der Längsachse, angebracht ist, wobei der röntgendichte Marker (172; 372) im Katheterlumen (113; 313) außermittig angeordnet ist; und
eine Ausströmdüse (40; 140; 240; 340; 440) im distalen Abschnitt, wobei die Ausströmdüse (40; 140; 240; 340; 440) proximal von der Einströmdüse (50; 150; 254, 256; 350; 450) so angeordnet ist, dass die Einströmdüse (50; 150; 254, 256; 350; 450) zwischen der Ausströmdüse (40; 140; 240; 340; 440) und dem Fluidstrahlausströmer (160; 360) angeordnet ist.

2. Katheter (10; 510) nach Anspruch 1, wobei sich die Einströmdüse (50; 150; 254, 256; 350; 450) um einen Abschnitt des Umfangs der Wand erstreckt, der durch einen Düsenbogen mit einer Mitte in einer Mitte des Katheterlumens in der Einströmdüse (50; 150; 254, 256; 350; 450) definiert ist und wobei die mehreren Fluidstrahlöffnungen (164; 364) im Fluidstrahlausströmer (160; 360) außerhalb des Düsenbogens liegen.

3. Katheter (10; 510) nach Anspruch 2, wobei ein Abschnitt des Fluidstrahlausströmers (160; 360) im Düsenbogen frei von Fluidstrahlöffnungen ist.

4. Katheter (10; 510) nach Anspruch 1, wobei sich die Hochdruckleitung (170; 370; 570) über die Einströmdüse (50; 150; 254, 256; 350; 450) hinweg erstreckt.

5. Katheter (10; 510) nach Anspruch 4, wobei die Hochdruckleitung (170; 370; 570) einen bogenförmigen Abschnitt (374) aufweist, der in der Einströmdüse (50; 150; 254, 256; 350; 450) angeordnet ist, wobei sich der bogenförmige Abschnitt (374) von einer Mitte des Katheterlumens (113; 313) in der Einströmdüse (50; 150; 254, 256; 350; 450) weg biegt.

6. Katheter (10; 510) nach Anspruch 5, wobei der röntgendichte Marker (172; 372) am bogenförmigen Abschnitt (374) der Hochdruckleitung (170; 370; 570) angebracht ist.

7. Katheter (10; 510) nach Anspruch 1, wobei der distale Abschnitt des Katheterkörpers zwischen der Einströmdüse (50; 150; 254, 256; 350; 450) und der Ausströmdüse (40; 140; 240; 340; 440) einen Bogen (480) bildet, wenn er unbeschränkt ist, und wobei die Einströmdüse (50; 150; 254, 256; 350; 450) und die Ausströmdüse (40; 140; 240; 340; 440) zu einer Mitte (481) des Bogens (480) weisen.

## Revendications

1. Cathéter de thrombectomie (10 ; 510) comprenant :
un corps de cathéter s'étendant à partir d'une partie distale (18 ; 118 ; 218 ; 318 ; 418) vers une partie proximale (16) le long d'un axe longitudinal (111 ; 311 ; 511) ;
un tube haute pression (170 ; 370 ; 570) s'étendant à travers une lumière du corps de cathéter, de la partie proximale (16) vers la partie distale (18 ; 118 ; 218 ; 318 ; 418), le tube haute pression (170 ; 370 ; 570) étant configuré pour se coupler avec une source de fluide à proximité de la partie proximale (16) et se terminant par un pulvérisateur de jet de fluide (160 ; 360) situé dans la lumière de cathéter (113 ; 313), le pulvérisateur de jet de fluide (160 ; 360) comprenant une pluralité d'ouvertures de jet de fluide (164 ; 364) configurée pour diriger une pluralité de jets de fluide à travers la lumière de cathéter (113 ; 313) vers la partie proximale (16), dans lequel le tube haute pression (170 ; 370 ; 570) s'étend le long d'un côté de la lumière de cathéter (113 ; 313) de sorte que le tube haute pression (170 ; 370 ; 570) est excentré dans la lumière de cathéter (113 ; 313) ;
un orifice d'entrée (50 ; 150 ; 254 ; 256 ; 350 ; 450) formé à travers une paroi du corps de cathéter dans la partie distale, dans lequel l'orifice d'entrée (50 ; 150 ; 254 ; 256 ; 350 ; 450) est situé de manière proximale par rapport au pulvérisation de jet de fluide (160 ; 360) ;
un marqueur radio-opaque (172 ; 372) fixé sur le tube haute pression (170 ; 370 ; 570) sur une longueur axiale de l'orifice d'entrée (50 ; 150 ; 254 ; 256 ; 350 ; 450) telle que mesurée le long de l'axe longitudinal, dans lequel le marqueur radio-opaque (172 ; 372) est excentré dans la lumière de cathéter (113 ; 313) ; et
un orifice de sortie (40 ; 140 ; 240 ; 340 ; 440) dans la partie distale, dans lequel l'orifice de sortie (40 ; 140 ; 240 ; 340 ; 440) est situé à proximité de l'orifice d'entrée (50 ; 150 ; 254, 256 ; 350 ; 450) de sorte que l'orifice d'entrée (50 ; 150 ; 254, 256 ; 350 ; 450) est situé entre l'orifice de sortie (40 ; 140 ; 240 ; 340 ; 440) et le pulvérisateur de jet de fluide (160 ; 360).

2. Cathéter (10 ; 510) selon la revendication 1, dans lequel l'orifice d'entrée (50 ; 150 ; 254, 256 ; 350 ; 450) s'étend autour d'une partie de la circonférence de la paroi définie par un arc d'orifice ayant un centre au niveau d'un centre de la lumière de cathéter dans l'orifice d'entrée (50 ; 150 ; 254, 256 ; 350 ; 450), et dans lequel la pluralité d'ouvertures de jet de fluide (164 ; 364) dans le pulvérisateur de jet de fluide (160 ; 360) est située à l'extérieur de l'arc d'orifice.

3. Cathéter (10 ; 510) selon la revendication 2, dans lequel une partie du pulvérisateur de jet de fluide (160 ; 360) à l'intérieur de l'arc d'orifice est dépourvue d'ouvertures de jet de fluide.

4. Cathéter (10 ; 510) selon la revendication 1, dans lequel le tube haute pression (170 ; 370 ; 570) s'étend sur l'orifice d'entrée (50 ; 150 ; 254, 256 ; 350 ; 450).

5. Cathéter (10 ; 510) selon la revendication 4, dans lequel le tube haute pression (170 ; 370 ; 570) comprend une partie arquée (374) située dans l'orifice d'entrée (50 ; 150 ; 254, 256 ; 350 ; 450), dans lequel la partie arquée (374) s'arque à l'opposé d'un centre de la lumière de cathéter (113 ; 313) à l'intérieur de l'orifice d'entrée (50 ; 150 ; 254, 256 ; 350 ; 450).

6. Cathéter (10 ; 510) selon la revendication 5, dans lequel le marqueur radio-opaque (172 ; 372) est fixé à la partie arquée (374) du tube haute pression (170 ; 370 ; 570).

7. Cathéter (10 ; 510) selon la revendication 1, dans lequel la partie distale du corps de cathéter entre l'orifice d'entrée (50 ; 150 ; 254, 256 ; 350 ; 450) et l'orifice de sortie (40 ; 140 ; 240 ; 340 ; 440) forme un arc (480) lorsqu'elle n'est pas retenue, et dans lequel l'orifice d'entrée (50 ; 150 ; 254, 256 ; 350 ; 450) et l'orifice de sortie (40 ; 140 ; 240 ; 340 ; 440) font face à un centre (481) de l'arc (480).
